# EUROPÄISCHE PATENTSCHRIFT

(11) **EP 1 087 793 B1**
(45) Veröffentlichungstag und Bekanntmachung des Hinweises auf die Patenterteilung: **15.02.2006**
(21) Anmeldenummer: 99926492.2
(22) Anmeldetag: 01.06.1999
(51) Int. Cl.: A61K 49/00, A61K 41/00, A61K 31/195, A61C 17/02, A61C 19/06, A61B 5/00, A61N 5/06

(54) **MATERIAL ZUR MODIFIZIERUNG DER OPTISCHEN EIGENSCHAFTEN UNTERSCHIEDLICHER ZELLEN UND GERÄT ZUM APPLIZIEREN DIESES MATERIALS**
MATERIAL FOR DIFFERENTLY MODIFYING THE OPTICAL PROPERTIES OF DIFFERENT CELLS AND DEVICE FOR APPLYING THIS MATERIAL
MATIERE DE MODIFICATION DIFFERENCIEE DES PROPRIETES OPTIQUES DE DIFFERENTES CELLULES ET DISPOSITIF POUR APPLIQUER CE MATIERE

(30) Priorität: 19.06.1998 DE 19827417
(43) Veröffentlichungstag der Anmeldung: 04.04.2001
(73) Patentinhaber: Karl Storz GmbH & Co. KG, 78532 Tuttlingen (DE)
(72) Erfinder: HAHN, Rainer, Dr., 72074 Tübingen (DE); IRION, Klaus, 78576 Liptingen (DE)
(74) Vertreter: Witte, Alexander
(86) Internationale Anmeldenummer: PCT/EP1999/003778
(87) Internationale Veröffentlichungsnummer: WO 2000/001350

(56) Entgegenhaltungen:
- EP-A- 0 049 905
- EP-A- 0 300 277
- EP-A- 0 743 029
- WO-A-93/21992
- WO-A-94/17797
- WO-A-95/07077
- WO-A-95/10243
- WO-A-96/39188
- WO-A-98/06456
- WO-A-98/09155
- WO-A-98/10711
- WO-A-98/30242
- DE-A- 2 725 793
- DE-U- 8 517 634
- DE-U- 29 705 934
- FR-A- 1 171 206
- US-A- 3 667 454
- US-A- 4 685 596
- US-A- 5 033 961
- US-A- 5 098 291
- US-A- 5 422 093
- US-A- 5 558 518
- US-A- 5 570 182
- US-A- 5 620 700
- LEUNIG, ANDREAS ET AL: "Fluorescence imaging and spectroscopy of 5- aminolevulinic acid induced protoporphyrin IX for the detection of neoplastic lesions in the oral cavity" AM. J. SURG., 1996, VOL. 172, NO. 6, PAGE(S) 674-677, XP002120195
- LEUNIG A. ET AL: "PHOTODYNAMISCHE DIAGNOSTIK VON NEOPLASIEN DER MUNDHÖHLE NACH LOKALER APPLIKATION VON 5-AMINOLÄVULINSÄURE" LARYNGO- RHINO- OTOLOGIE, 1996, VOL. 75, NO. 8, PAGE(S) 459-464, XP002120196
- VONARX V ET AL: "Potential efficacy of a delta 5-aminolevulinic acid bioadhesive gel formulation for the photodynamic treatment of lesions of the gastrointestinal tract in mice." J PHARM PHARMACOL, JUL 1997, VOL. 49, NO. 7, PAGE(S) 652-6, XP002078605 ISSN: 0022-3573
- MESSMANN H ET AL: "Photodynamische Diagnostik gastrointestinaler Prakanzerosen nach Sensibilisierung mit 5-Aminolavulinsaure. Eine Pilotstudie." DTSCH MED WOCHENSCHR, 24-4-1998, VOL. 123, NO. 17, PAGE(S) 515-21, XP002120197
- ROPER J.M. ET AL: "Tetrapyrrole biosynthesis in several haem-dependent anaerobic pathogens" REVIEWS IN MEDICAL MICROBIOLOGY, 1997, VOL. 8, NO. SUPPL. 1, PAGE(S) S13-S17, XP002120198
- BAUMGARTNER R ET AL: "Inhalation of 5-aminolevulinic acid: a new technique for fluorescence detection of early stage lung cancer." J PHOTOCHEM PHOTOBIOL B, NOV 1996, VOL. 36, NO. 2, PAGE(S) 169-74, XP002120199 ISSN: 1011-1344
- WEBBER J ET AL: "On-line fluorescence of human tissues after oral administration of 5-aminolevulinic acid." J PHOTOCHEM PHOTOBIOL B, APR 1997, VOL. 38, NO. 2-3, PAGE(S) 209-14, XP002120200 ISSN: 1011-1344
- PENG Q ET AL: "5-Aminolevulinic acid-based photodynamic therapy. Clinical research and future challenges." CANCER, JUN 15 1997, VOL. 79, NO. 12, PAGE(S) 2282-308, XP002120201 ISSN: 0008-543X
- ACKERMANN G ET AL: "Simulations on the selectivity of 5-aminolaevulinic acid-induced fluorescence in vivo." J PHOTOCHEM PHOTOBIOL B, DEC 1998, VOL. 47, NO. 2-3, PAGE(S) 121-8, XP002120202 ISSN: 1011-1344
- W.E. GRANT ET AL.: "Photodynamic therapy of oral cancer: photosensitisation with systemic aminolaevulinic acid" THE LANCET, Bd. 342, 17. Juli 1993 (1993-07-17), Seiten 147-148, XP002132811 LANCET LIMITED. LONDON., GB ISSN: 0140-6736
- DATABASE WPI Section PQ, Week 199834 Derwent Publications Ltd., London, GB; Class P34, AN 1998-396648 XP002132812 -& RU 2 101 047 C (PROKHONCHUKOV A A), 10. Januar 1998 (1998-01-10)
- DATABASE WPI Section Ch, Week 199424 Derwent Publications Ltd., London, GB; Class B05, AN 1994-197879 XP002132813 & SU 1 792 714 A (DNEPR MED INST), 7. Februar 1993 (1993-02-07)
- DATABASE WPI Section PQ, Week 197505 Derwent Publications Ltd., London, GB; Class P34, AN 1975-B2816W XP002132814 & SU 405 555 A (ALMA-ATAI MEDICAL INST), 5. Juli 1974 (1974-07-05)

## Beschreibung

Die Erfindung betrifft die Verwendung eines Materials zur unterschiedlichen Modifizierung der optischen Eigenschaften unterschiedlicher Zellen gemäß Anspruch 1, sowie ein Gerät zum Applizieren eines solchen Materiales gemäß dem Oberbegriff des Anspruches 17.

Paradontose ist eine verbreitete Krankheit des Zahnhalteapparates. Paradontopathien werden durch in Zahnfleischtaschen lokalisierte (subgingivale) Bakterien verursacht. Es handelt sich dabei teilweise um adhärent an die Zahnwurzeloberfläche gebundene zumeist grampositive Bakterien, die zu Konkrementen (subgingivaler Zahnstein) verkalken, zum anderen um dem Taschenweichgewebe zugewandte nicht adhärente zumeist gramnegative Bakterien, die z.B. im Teil im Taschenfluid motil sind. Gerade diese motilen Bakterien spielen bei der Progression einer Paradontitis eine wesentliche Rolle.

Im Zuge der Progression der Paradontitis können die Bakterien das Taschenepithel durchwandern und in das subepitheliale Bindegewebe eindringen, so daß sie das entzündliche Infiltrat umgeben. Es kommt zu komplizierten Wechselwirkungen mit der an dieser Stelle massiv etablierten Immunabwehr des erkrankten Menschen, welche durch (Mikro)Negrosen, eitrige Abzesse oder als Reaktion auf die Immunwechselwirkung z.B. durch Aktivierung knochenabbauender körpereigener Zellen zum Verlust von paradontalem Stützgewebe und Ausbildung bzw. Vertiefung einer Zahnfleischtasche und/oder der Retraktion der Gingivaweichgewebe führt. Besondere Bedeutung kommt dabei Prozessen zu, die in der Tiefe der Tasche oder im Problembereichen wie z.B. der Wurzelfurkation ablaufen.

Zur Keimreduktion in den Taschen werden bisher mechanische Reinigungstechniken (z.B. Scaling, Kürettagen, Reinigung mit Ultraschallinstrumenten) oder einfache Taschenspülungen empfohlen. Ein systemische Anwendung von Antibiotika ist mit erheblichen Nebenwirkungen belastet, zum einen wegen des breiten Spektrums der ursächlichen Bakterien, zum anderen wegen der Lokalisation der Bakterien außerhalb des Blutkreislaufes. Lokale Anwendungstechniken durch Applikation der Antibiotika direkt in den Zahnfleischtaschen sind in ihrer Wirkung oftmals unsicher, weil die Diffusion in alle Taschenbereiche nicht ausreichend ist oder die Deposition nicht lange genug dauert oder die Höhe der Wirkstoffkombination nicht ausreicht. Daher werden Antibiotika üblicherweise nur als unterstützende Maßnahme zu herkömmlichen zumeist mechanischen Verfahren angewendet.

Wegen der komplexen Geometrien der befallenen Parodontien oder Zahnfleischtaschen ist der Zugang zu den kranken Gewebebereichen behindert, und die angestrebte Keimreduktion wird oft nicht erreicht. In der Folge kommt es nach unterschiedlichen Zeitintervallen in Abhängigkeit von der zumeist angetroffenen immunologischen Prädisposition des Patienten zu einer Wiederbesiedlung einer zuvor therapierten Tasche mit einem Rezidiv der Erkrankung.

Besonders problematisch ist die Keimreduktion im Bereich des infiltrierten Taschenepithels und des angrenzenden Bindegewebes.

Vorraussetzung für eine verbesserte Therapie ist zunächst, über den Ist-Zustand der Erkrankung genauere Information zu haben, welche eine bessere Voraussage über die Weiterentwicklung der Erkrankung, insbesondere das Auftreten akuter Erkrankungsschübe gestattet. Bisher kann lediglich im nachhinein anhand von Eiterentleerungen aus der Tasche eine zuvor aktive Tasche identifiziert werden. Wenn diese Identifizierung möglich ist, ist jedoch der Verlust des Stützgewebes bereits eingetreten. Neuerdings zur Diagnostik einzelner Bakterien in Zahnfleischtaschen eingesetzte Bakterien-Gentests sind teuer und benötigen mehrere Tage zur Auswertung. Aus diesen Gründen eignen sie sich nicht für Routineanwendungen. Auch kann aus derartigen Tests nur auf die Genome der Bakterien zurückgeschlossen werden. Eine Unterscheidung nach Stoffwechselaktivitäten der Bakterien, die für die Paradontalerkrankung kausal sind, ist aber nicht möglich.

Aus dem Dokument Vonarx V. et al., J.Pharm.Pharmacol 49, (1997), 652-656 sind Gele als Grundsubstanz in Form von Polyacrylsäure, Polysacchariden, Polyalkylenoxid-Copolymeren bekannt, die 5-Aminolävulinsäure (ALA) enthalten. Derartige Gele werden für die photodynamische Diagnostik im Gastrointestinaltrakt eingesetzt.

Das Dokument Baumgartner R. et al., J.Photochem Photobiol.B: Biology 36, (1997), 169-174 beschreibt im Abstract, daß Cremes bekannt sind, die die Modifikationssubstanz ALA enthalten, und die in der Hautkrebstherapie eingesetzt werden.

Die WO 94 17797 beschreibt die Verwendung einer photoaktiven zytotoxischen Verbindung, z.B. ALA, die in Kombination mit einem Trägermaterial eingesetzt wird, welche topisch angewendet werden.

Die WO 98 09155 beschreibt eine Methode zum Nachweis von Hautkrebs, bei der ALA in einer Konzentration von 5 bis 25 % in einer Ö/W- oder W/Ö-Emulsion, also einer viskosen Flüssigkeit, eingesetzt und einer UV-Bestrahlung unterworfen wird.

Das Dokument Peng, Q. et al., Cancer, Vol. 79, Nr. 12, (1997), 2282-2308 beschreibt eine Ö/W-Emulsion von ALA sowie eine Creme, die 2 bis 5 % ALA enthält, die lokal an die Haut verabreicht wird.

Die US-A-5 620 700 beschäftigt sich mit einer Spritze, die ein Material in die Zahnfleischtasche appliziert. Diese Spritze besteht aus einer Hohlnadel, einer Kartusche, welche das Material enthält und einem Presskolben.

Die US-A-5 558 518 stellt eine Applikationappatur in Form einer Spritze vor, bestehend aus einem komprimierbaren Kolben, der die zu applizierende Flüssigkeit enthält und einer Nadel. Die Nadel verjüngt sich dabei zu ihrer Spitze hin.

Die US-A-4 685 596 beschreibt ein Abgabegerät, das aus einer Kanüle und aus einem Zylinder besteht, in welchem sich die abzugebende Flüssigkeit befindet. Die kontrollierte Abgabe dieser Flüssigkeit erfolgt durch die Freisetzung eines Treibgases, das den Kolben in den Zylinder drückt.

Die EP-A-0 300 277 beschreibt eine Vorrichtung, die eine Lösung von einem Arzneimittel unter Druck aus einem Zylinder durch eine sich verjüngende Kanüle appliziert.

Die US-A-5 098 291 beschreibt ein Gerät, das eine unter Druck stehende Materialquelle aufweist.

Die US-A-5 033 961 stellt ein Applikationsgerät vor, das die zu applizierende Flüssigkeit aus einem Reservoir unter Druckanwendung in eine Kanüle fördert.

Die Erfindung widmet sich daher dem Problem, auf einfache Weise, zu geringen Kosten und rasch Informationen über das Ausmaß einer Paradonditis zu erlangen, insbesondere auch Paradonditis im Anfangsstadium sicher erkennen zu können.

Erfindungsgemäß wird dies durch die Verwendung eines Materials zur unterschiedlichen Modifizierung der optischen Eigenschaften unterschiedlichen Zellen mit einem Grundmaterial und einer in diesem verteilten Modifikationssubstanz bei der Herstellung eines Diagnostikums und/oder Therapeutikums für die Paradontologie und/oder Kariesdiagnostik gelöst, wobei das Grundmaterial eine viskose Flüssigkeit, ein Gel, ein flächiges oder dreidimensionales poröses Substrat oder ein in situ erhärtendes Material, insbesondere ein Film bildendes Material umfaßt und die Modifikationssubstanz die Menge an im HÄM-Zyklus gebildetem Protoporphyrin erhöht, wobei die Modifikationssubstanz entweder ein Substrat für Enzyme, die nach der 5-Aminolävulinat-Synthase bis zum Schritt der Synthese des Protoporphyrins aktiv sind, oder ein Antagonist der Protoporphyrin abbauenden Ferro-Chelatase oder eine Eisen inaktivierende Substanz enthält.

Die Aufgabe wird ferner durch ein Gerät zum Applizieren eines solchen viskosen Modifikationsmaterials gelöst.

Materialien, die unterschiedliche Zellen in einer für sie typischen Eigenschaft unterschiedlich beeinflussen, sind auf anderen Gebieten der Medizin bekannt. So werden z.B. radioaktive Tracer zur Erkennung von Krebszellen verwendet, da sie sich aufgrund des höheren Stoffwechselumsatzes in diesen bevorzugt ablagern. Andere Modifikationssubstanzen sind solche, die direkt in den Zellstoffwechsel eingreifen, genauer gesagt in die Porphyrinbiosynthese. Die Verabreichung geeigneter Modifikationssubstanzen, die weiter unten genauer beschrieben werden, führt dazu, daß sich das Fluoreszenzspektrum stoffwechselaktiver Zellen verstärkt. Hierdurch können diese Zellen auch quantitativ und in ihrer räumlichen Verteilung bestimmt werden, und auf diese Information kann dann die Planung der Therapie erfolgen.

Beispiele für ein derartiges Vorgehen sind aus den US-Patentschriften 5 422 093, 5 234 940, 5 211 938 und 5 079 262 ersichtlich. Es geht dabei um die Erkennung und anschließende Behandlung maligner und nichtmaligner Gewebeabnormalitäten unter Einsatz von Aminolävulinsäure. Diese wird in Form einer wässrigen Wirkstofflösung verwendet. Regt man die zellfluoreszenz im violetten Bereich (375 nm bis 440 nm) an, beobachtet man eine rote Fluoreszenz. Durch Verwendung eines Beobachtungsfilters wird das diffus reflektierte blauviolette Anregungslicht ausgeblendet, und man sieht die stoffwechselaktiven Gewebebereich rot vor leicht grünlich erscheinendem Hintergrund (Eigenfluoresenz des gesunden Gewebes).

Die Geschwindigkeitskonstante, mit welcher der Stoffwechsel der Zellen abläuft, macht es erforderlich, daß der Wirkstoff über eine längere Zeit (mindestens 15 Minuten, typischerweise 120 Minuten) im wesentlichen unverändert im Kontakt mit dem Gewebe verbleibt.

Es wurde nun erkannt, daß man die auf einem anderen Gebiet der Medizin entwickelte Technik zur Erkennung kranker Zellen auch auf dem Gebiet der Paradontitis einsetzen kann, wenn man ein Grundmaterial verwendet, welches sich nicht schnell mit Speichel vermischt und somit die Modifikationssubstanz am gewünschten Ort hält. Hierfür kommen erfindungsgemäß in Frage ausreichende Viskosität aufweisende Flüssigkeiten, Gele, flache poröse Substrate wie Gewebe, Vliese und dergleichen oder auch dreidimensionale poröse Substrate, die in ihren Hohlräumen Partikel der Modifikationssubstanz oder eine konzentrierte Lösung derselben aufnehmen können und so die Modifikationssubstanz über einen längeren Zeitraum verteilt freisetzen. Eine weitere Alternative besteht in in situ erhärtenden Materialien wie Filmbildnern, welche die Modifikationssubstanz an der Oberfläche binden oder einschließen.

Vorteilhafte Weiterbildungen der Erfindung sind in Unteransprüchen angegeben.

Die weiterbildung der Erfindung gemäß Anspruch 2 gewährleistet zum einen, daß sich das Grunämaterial unter den am Einsatzort auf es einwirkenden Bedingungen stabil bleibt, und darüber hinaus die zu untersuchende Umgebung nicht verändert.

Die Weiterbildung der Erfindung gemäß Anspruch 3 erlaubt ein leichtes Applizieren des Materiales, gewährleistet aber trcizdem, daß das Material nach Applizieren im erforderlichen Ausmaße formstabil ist.

Auch die Weiterbildung der Erfindung gemäß Anspruch 4 ist im Hinblick auf gute Formstabilität des Materiales nach Applikation von Vorteil.

Die gleiche Wirkung erhält man gemäß Anspruch 5.

Ein Material, wie es in Anspruch 6 angegeben ist, eignet sich besonders gut zum Füllen von Zahntaschen ohne nennenswerte Verdrängung von Flüssigkeit aus den Zahntaschen. Man kann auf diese Weise den Zustand einer Zahntasche vor Behandlung besonders gut diagnostizieren.

Die Weiterbildung der Erfindung gemäß Anspruch 7 gestattet es, zumindest durch dünne Schichten des Materiales hindurchzusehen und Anregungslicht durch das Material hindurchzuschicken.

Grundmaterialien, wie sie im Anspruch 8 angegeben sind, eignen sich zur in situ Anbringung einer besonders gut formstabilen Materialschicht.

Verwendet man ein Material gemäß Anspruch 9, so kann man diejenige Zeit, innerhalb welcher das Material am Einsatzort verbleiben muß, zugleich zu Therapiezwecken verwenden. Typische Zeiten, die zwischen der Applikation des Materiales und der Diagnostizierung der durch das Material modifizierten Zellen liegen, betragen etwa zwei Stunden. Man erhält in dieser Wartezeit somit auch einen guten Therapieeffekt.

Die Weiterbildung der Erfindung gemäß Anspruch 10 ist im Hinblick auf gute Stabilität des Materiales im Mundmilieu von Vorteil, und durch ein solches Material wird auch bezüglich des pH-Wertes der Ausgangszustand der zu untersuchenden Zellen bzw. Gewebebereiche nicht nennenswert geändert.

Grundmaterialien, wie sie im Anspruch 11 angegeben sind, sind im dentalen Bereich seit langer Zeit erprobt und zeichnen sich durch hervorragende Formstabilität und gute Formanpassung aus. Für Untersuchungen an Gewebebereichen, die gut zugänglich sind, bietet ein solches Material gute Dienste.

Die weiterbildungen der Erfindung gemäß den Ansprüchen 12 und 13 erlauben das Erkennen kranker Zellen einfach mit dem Auge oder unter Verwendung einer Kamera. Man kann auf diese Weise die Intensität der Erkrankung und die räumliche Verteilung der kranken Gewebebereiche einfach mit dem Auge oder einer Kamera feststellen. Ein solches direktes optisches Bild der Erkrankung ist für das Planen einer Therapie besonders hilfreich.

Die Beeinflußung des Häm-Stoffwechsels, wie dies im Anspruch 14 vorgeschlagen wird, ist für ein breites Spektrum unterschiedlicher Zellen besonders aussagekräftig und signifikant. Die Weiterbildung der Erfindung gemäß Anspruch 14 hat auch den Vorteil, daß derartige Modifikationsmittel von den eingangs angesprochenen anderen Gebieten der Medizin (Erkennung canceröser Gewebe) her zu verfügung stehen. Die entsprechenden Substanzen können verhältnismäßig preisgünstig synthetisiert werden.

Durch das im Anspruch 15 angegebene Modifikationsmittel wird der Einbau von Eisen in Protoporphyrin modifiziert, also in die letzte Vorstufe des Häm.

Anspruch 16 nennt bevorzugte Konzentrationen der Modifikationssubstanz, welche im Hinblick auf ausgeprägte Modifikation einerseits und nicht zu starke Änderung des Zellmilieus vorteilhaft sind.

Ein Gerät, wie es im Anspruch 17 angegeben ist, erleichert das Applizieren von viskösem oder breiigem Material.

Bei einem Gerät gemäß Anspruch 18 kann man mit der Applikations-Kanüle zugleich die Tiefe einer Zahntasche messen und bei bekannter Zahntaschentiefe ablesen, wie weit die Kanüle schon in die Zahntasche hineinbewegt worden ist.

Bei verwendung eines Gerätes gemäß Ansprüchen 19 erfolgt die Zufuhr des Materiales in die Zahntasche so schonend, daß die Ausgangsverhältnisse nur wenig gestört werden.

Mit einem Gerät gemäß Anspruch 20 kann man das Material durch die Gingiva hindurch zu der Tascheninnenfläche benachbarten Gewebebereichen bringen.

Die Weiterbildung der Erfindung gemäß Anspruch 21 ist im Hinblick auf eine besonders feinfühlige Materialdosierung von Vorteil.

Vom eingangs angesprochenen Gebiet der Behandlung maligner und nichtmaligner Gewebeabnormalitäten ist an sich auch bekannt, daß durch die dort verwendeten in den Porphyrinstoffwechsel eingreifenden Modifikationssubstanzen eine Fotosensibilisierung bevorzugt der kranken Zellen erfolgt. Diese Fotosensibilisierung kann man dazu nutzen, durch intensive Einstrahlung von Licht geeigneter Wellenlänge diese kranken Zellen so zu schädigen, daß sie absterben. Derartige Verfahren werden insbesondere zur Behandlung von Blasenkarzinomen, Hirntumoren oder Mundhöhlenkarzinomen verwendet.

Nachstehend wird die Erfindung anhand von Ausführungsbeispielen unter Bezugnahme auf die Zeichnung näher erläutert. In dieser zeigen:
- Figur 1:: einen Ausschnitt aus dem Häm-Kreislauf, anhand dessen die Photosensibilisierung von Zellen erläutert wird;
- Figur 2:: einen axialen Schnitt durch einen Zahn und benachbarte Weichgewebe sowie eine seitliche Ansicht eines Applikators für Material, durch welches eine Fotosensibilisierung kranker Zellen bzw. von Bakterien erfolgt;
- Figur 3:: eine ähnliche Darstellung wie Figur 2, wobei jedoch ein abgewandelter Applikator wiedergegeben ist;
- Figur 4:: einen axialen Schnitt durch einen nochmals abgewandelten Applikator, ähnlich zu demjenigen nach Figur 2; und
- Figur 5:: eine Dichthaube, durch welche in eine Zahnfleischeingebrachtes Material zur Modifizierung der optischen Eigenschaften kranker Zelle während der Einwirkungszeit gegen das Mundmilieu abgedichtet wird.

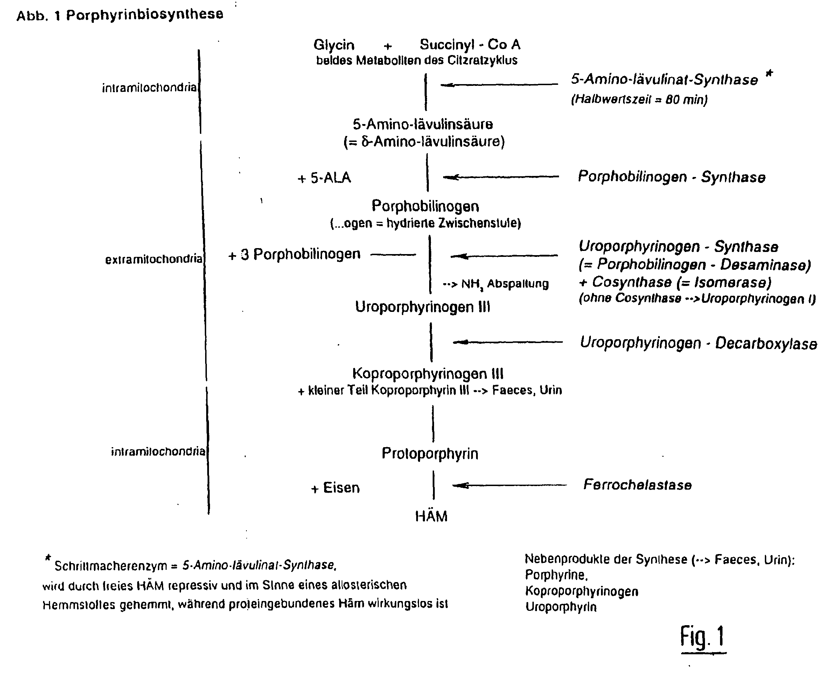

Figur 1 zeigt einen Ausschnitt der Porphyrinbiosynthese in einer eukaryontischen Zelle. Die fluoreszierende Substanz, welche Zellen mit stärkerem Stoffwechsel (in der Regel kranke Zellen) von normalen Zellen zu unterscheiden gestattet, ist das vor dem letzten Syntheseschritt vorliegende Protoporphyrin. Eine Erhöhung der in einer Zelle enthaltenen Menge an Protoporphyrin kann man zum einen dadurch erreichen, daß man durch Erhöhung der Konzentration des Ausgangsstoffes einer Vorstufe die Protoporphyrin-Bildung erhöht, oder dadurch, daß man den Protoporphyrinabbau reduziert, indem man die Menge des für den letzten Syntheseschritts zur verfügung stehenden Eisens reduziert. Substanzen, die in diesem Sinne wirken, werden in den Patentansprüchen und nachstehend als Modifikationssubstanzen angesprochen.

Bei dem aus Figur 1 ersichtlichen Teil des Häm-Zyklus stellt die im ersten dargestellten Syntheseschritt mitwirkende 5-Amino-Lävulinat-Synthase ein Schrittmacherenzym dar. Dies bedeutet, daß dieses Enzym die Gesamtgeschwindigkeit des nachfolgenden Teiles der Synthese steuert. Aus diesem Grunde scheiden andere Reaktionsteilnehmer, die nur an in Figur 1 nicht wiedergegebenen früheren Syntheseschritten teilnehmen, als Modifikationssubstanzen aus. Dagegen sind diejenigen Reaktionsteilnehmer geeignet, die an demjenigen Teil der Synthesekette teilnehmen, der zwischen 5-Amino-Lävulin-Säure und Protoporphyrin liegt (diese Substanzen eingeschlossen).

Die einzelnen Syntheseschritte sind aus Figur 1 im einzelnen erkennbar, ebenso die an ihnen beteiligten Moleküle. Insoweit kann auf eine detaillierte Beschreibung von Figur 1 verzichtet werden. Hinzuweisen ist noch darauf, daß die 5-Amino-Lävulinat-Synthase durch freies Häm repressiv und durch allosterische Hemmung (also doppelt) in seiner Wirmung gehemmt wird. Proteingebundenes Häm hat dagegen keine solche Hemmfunktion. Auf diese Weise regelt sich der Hämzyklus automatisch. Die Schrittmacherfunktion der 5-Amino-Lävulinat-Synthase ergibt sich aus der vergleichsweise kurzen Halbwertszeit von nur 80 Minuten.

Derzeit bevorzugt wird als Modifikationssubstanz das kommerziell problemlos erhältliche synthetisierte 5-Amino-Lavulin-Säure-Hydrochlorid (Merck, Art.-Nr. 124802-0500; L 326102).

Es versteht sich aus den obigen Darlegungen, daß man die wartezeit zwischen der Applikation des die Modifikationssubstanz enthaltenden Materiales, die bei Aminolävulinsäure bei ca. 2 Stunden liegt, bis die Diagnostik erfolgen kann, dann verkürzt werden kann, wenn man fortgeschrittenere Stoffwechselzwischenprodukte der Synthese verwendet, z.B. Porphobilinogen, Uroporphyrinogen III oder Koporphyrinogen III. Gut geeignet ist auch Uroporphyrinogen I oder dessen metabolische Vorstufen, welches zu einer ausgeprägteren Fotosenibilisierung führt und sich daher besonders auch für Therapiezwecke eignet.

Weiter verwendbare Modifikationssubstanzen sind Agonisten und/oder Antagonisten der Enzyme des Stoffwechsels. Zum Beispiel fördert ein Cosynthase-Hemmstoff unter gleichzeitiger Gabe der Ausgangssubstanzen, z.B. der Amino-Lävulin-Säure, die Bildung des Stoffwechselmetaboliten Uroporphyrinogen I mit nachfolgenden modifzierten Stoffwechselprodukten. Man erhält so eine verbesserte Fotosensibilisierung der Bereiche, welche vermehrt diese Stoffwechselprodukte einlagern.

Als den Abbau des Protoporhyrins beeinflußende Substanzen sind Ferrochelastase-Antagonisten oder Eisenkomplexbildner wie Deferoxamin zu nennen. Diese erhöhen die Menge des in der Fluoreszenz erscheinenden Protoporphyrins bei definierter Zugabe der anderen Stoffwechselvorprodukte (ggf. auch zusätzlich zu einer durch andere Substanzen erhöhten Bildung des Protoporhyrins).

Nachdem oben stehend Einzelheiten zu den verwendbaren Modifikationssubstanzen dargelegt sind, wird nachstehend das Grundmaterial näher erläutert:

Das Grundmaterial hat die Aufgabe, die Modifikationssubstanz in der Nähe des zu behandelnden Gewebebereiches bzw. der zu modifizierenden Zellen zu halten. Aus diesem Grunde soll erfindungsgemäß das Grundmaterial eine Formstabilität aufweisen. Dabei ist nicht an eine Formstabilität im Sinne fester Körper oder aus elastomerem Material gefertigter Körper gedacht, sondern an eine Formstabilität, die besser ist als die von niederviskösen Flüssigkeiten wie Wasser, Alkohol, usw. In diesem Sinne sind formstabile Materialien auch ölähnliche Substanzen oder Gele.

Dabei haben hydrophile Grundmaterialien der Vorteil, daß sie sich gut mit dem Zahnhals oder der Tascheninnenseite verbinden, während hydrophobe Materialien schwebend in der Taschenflüssigkeit gehalten werden bzw., wenn es sich um ölähnliche Materialien handelt, die Sulcus-Flüsverdrängen können, falls dies erwünscht ist, um nur die an dem Zahnhals bzw. der Innsenseite der Gingiva haftenden Bakterien zu diagnostizieren.

Eine erste Gruppe von geeigneten Grundmaterialien sind Hohlräume aufweisende Substrate. In deren Hohlräumen kann die Modifikationssubstanz selbst oder eine Lösung der Modifikationssubstanz in einem polaren oder nichtpolaren Lösungmittel aufgenommen werden, wobei man die Viskosität etwa verwendeter Lösungen auf die Struktur der Hohlräume des Substrates und auf die chemische Natur des Substratmateriales im Hinblick auf die gewünschte Länge der Modifikationssubstanzen-Abgabezeit wählt.

Beispiele für dreidimensionale offenporige Substrate sind z.B. Substrate, welche durch Anordnungen kleiner Röhrchen gebildet sind, Strukturschäume, Sintermaterialien (auch aus organischen Materialen wie PTFE) usw.

Beispiele für flächige offenporige Substrate sind Gewebe, Gewirke, vliese, Fadengelege.

In diese offenporigen Substrate wird die Modifikationssubstanz, falls sie in Lösung vorliegt, durch Kapilarwirkung und/oder Druckeinwirkung eingebaut. Liegt die Modifikationssubstanz in fester Form (z.B. Puder) vor, so kann man sie durch mechanisches Einarbeiten (z.B. Einwalzen mechanisch mit dem offenporigen Substrat verbinden, wobei sich die Partikel der Modifikationssubstanz mit dem Substrat mechanisch verhaken. Man kann in Puderform vorliegende Modifikationssubstanz auch mit puderförmigem Grundmaterial verpressen oder unter Verwendung eines Bindemittels verbinden.

Eine zweite Gruppe von Substraten haben eine geschlossene Oberfläche. Beispiele sind Kunststoffolien, z.B. Polyethylenfolien. Weitere Beispiele für flächige Substrate sind membranartige Gebilde.

Bei Ihnen kann man das Verbinden der Partikel der Modifikationssubstanz mit der Unterlage unter Verwendung von unter Einsatzbedingungen zumindest vorübergehend beständigen Klebemitteln vornehmen oder auch ein erweichbares, z.B. aus thermoplastischem Material gefertiges Substrat kurzfristig aufwärmen, so daß es in einen klebrigen Zustand kommt, und das in Pulverform vorliegende Material der Modifiaktionssubstanz auf die klebrige Oberfläche blasen. Analog kann man bei anderen Kunststoffmaterialien die Oberfläche des Substrates durch chemisches Anlösen in klebrigen Zustand bringen und dann ebenfalls Puder als Modifikationssubstanz auf die Oberfläche leiten.

Eine weitere Gruppe von Grundmaterialien sind wasserlösliche Gele. In diese kann die Modifikationssubstanz, auch in hoher Konzentration eingebaut werden.

Wichtig ist, daß solche Gele nicht vorschnell vor Abgabe der Modifikationssubstanz und vor Ablauf der notwendigen Stoffwechsel-Einwirkzeit aufgelöst und aus einer Zahnfleischtasche herausgespült wird. Das Gel (alle seine Bestandteile außer der Modifikationssubstanz) sollten keine die Stoffwechselaktivität oder Vitalität von Bakterien oder Abwehrzellen beinträchtigende Wirkung haben.

Es können anorganische Gele (Gelierung durch Zugabe von thixotropen Füllstoffen wie pyrogenes Siliciumdioxid oder Aluminiumdioxid) und/oder organische Gele (z.B. Hydroxyethylcellulosegel etc.) verwendet werden. Auch Substanzen wie modifiziertes Wasserglas sind verwendbar. Bevorzugt sind thixotrope Gele auf (anorganischer) Glyzerinbasis.

Weitere Grundmaterialien sind in situ erhärtende Materialien, insbesondere lack- oder filmbildende Substanzen, welche die Modifikationssubstanz enthalten und in situ aufgetragen werden, wo sie einen gegen das Umgebungsmilieu beständigen Film bilden.

Eine weitere Alternative besteht darin pulverförmige Modifikationssubstanz oder eine Modifikationssubstanzlösung in einen Mikrobeutel mit permeablen Wänden zu geben.

Das aus Grundmaterial und Modifikationssubstanz bestehende Modifikationsmaterial enthält die Modifikationssubstanz in einem Anteil von etwa 0,5 Gewichtsprozent bis hin zu etwa 50 Gewichtsprozent. Bevorzugt sind Konzentrationen zwischen 5 Gewichtsprozent und 40 Gewichtsprozent, nochmals bevorzugt zwischen 10 und 20 Gewichtsprozent.

Den oben beschriebenen Modifikationsmaterialien ist gemeinsam, daß sie ohne zusätzliches Eintragen wesentlicher Flüssigkeitsmengen am Einsatzort appliziert werden können, so daß sie in den Zahnfleischtaschen angefundene motile Bakterien nicht herausspülen und man so die unverfälschten Ausgangsbedingungen messen kann. Durch Diffusion verteilt sich die Modifikationssubstanz gleichmäßig in den Zahnfleischtaschen bis hin zum Taschenboden und in die Interradikulärbereiche. Bei der oben angesprochenen Zusammensetzung des Modifikationsmateriales wird auch das in der Zahnfleischtasche vorliegende Milieu nicht oder nur wenig beeinträchtigt.

Nachstehend wird nun der Ablauf einer Diagnose näher beschrieben, bei welcher ein Modifikationsmaterial vom Geltyp verwendet wird.

Bei der Erstdiagnostik eines Patienten im Hinblick auf eine Paradontalerkrankung oder bei einer Befundkontrolle im Sinne eines Monitoring werden zunächst die Taschentiefen um die einzelnen Zähne zumeist jeweils an verschiedenen Stellen und auch im Bereich etwaiger Zahnfurkationen gemessen. Hierzu wird eine Parodontalsonde verwendet, welche mit definiertem Anpressdruck bis zum Taschenfundus in die Tasche eingeführt wird. Unmittelbar nach dem Entfernen der Sonde werden die Befunde "Blutung auf Sondierung" und "Eiteraustritt" erhoben und mit dem Grad der Entzündung korreliert. Bei diesem klassischen Vorgehen wird der Inhalt der Zahntasche durch das Sondieren geringfügig geändert. Wie man dies vermeiden kann, wird weiter unten noch genauer beschrieben.

Um Inaktivierungen durch Lichtexposition oder Wechselwirkungen mit dem hydrolytischen Gel zu vermeiden, wird das Gel erst kurz vor Anwendung gemischt und mit der Modifikationssubstanz zusammengegeben. Das Gel wird auf einen pH-wert im leicht saueren bis neutralen Bereich eingestellt, z.B. im Bereich zwischen pH 4,5 und pH 7,5. Als besonders vorteilhaft hat sich eine pH-Einstellung auf etwa 5 erwiesen.

Zur Vermeidung von Inaktivierungen während der Lagerzeit bzw. im Hinblick auf eine Verlängerung der Lagerzeit wird die Modifikationssubstanz getrennt vom Grundmaterrial und ggf. in einer Pufferlösung (z.B. Zitratpuffer) aufbewahrt. Eine vorteilhafte Möglichkeit der Aufbewahrung ist die in einer Zwei-Kammer-Patrone, die unmittelbar vor Gebrauch durch Perforation der Zwischenwand zwischen den beiden Kammern aktiviert wird. Dann werden die beiden Komponenten miteinander vermischt, z.B. in einem mechanischen Rüttelmischer, wie er in Zahnarztpraxen vorhanden ist. Alternativ kann in Pulverform vorliegende Modifikationssubstanz auch auf einer sterilen Platte mit dem Grundmaterial gemischt werden und anschließend z.B. mit einer sterilen Spatel in einen Applikator oder Dispenser eingebracht werden. Die letztgenannte Art des Vorgehens ermöglicht es dem Anwender, die Menge der in das Modifikationsmaterial eingearbeiteten Modifikationssubstanz nach seinen jeweiligen Bedürfnissen unterschiedlich zu wählen.

Das so hergestellte Modifikationsmaterial kann dann mit einem stabförmigen Werkzeug in die Tasche eingebracht und dort verteilt werden, vorzugsweise erfolgt die Applikation mit einer Kanüle.

Man läßt dann das Modifikationsmaterial etwa zwei Stunden auf die zu untersuchenden Zellen einwirken. Es wird dort verstoifwechselt, und zwar, wie oben dargelegt, stärker in stoffwechselaktiven Zellen.

Nach dieser Einwirkungszeit wird der Untersuchungsbereich mit ein Anregungslicht (im Fall von 5-Amino-Lävulin-Säure violettem Anregungslicht) bestrahlt, welches man z.B. durch Ausfiltern den entsprechenden Wellenbereiches aus dem Licht einer Weißlichtquelle (z.B. Halogenlampe, Xenonlampe) erstellt. Unter Verwendung eines imoten liegenden Filters für das Meßlicht wird die vom Anregungslicht erzeugte Fluoreszenz beobachtet, entweder direkt oder unter Verwendung einer Fernsehkamera. Auf diese Weise erhält man die Information über die Menge und Verteilung kranker Zellen in der Zahntasche und damit über das Ausmaß der Paradontitis. Man lernt so, wo besonders betroffene Gewebebereiche liegen und kann diese bei der sich anschließenden Therapie in besonderer Weise berücksichtigen.

Anschließend werden noch vorhandene Rückstände des Gels und von diesem freigegebene Modifikationssubstanzen aus der Tasche herausgespült.

Ein weiteres Ausführungsbeispiel kann darin bestehen, daß man z.B. Papier mit einer Lösung der Modifikationssubstanz tränkt und das getränkte Papier vorsichtig in die Zahntasche schiebt. Die Modifikationssubstanz wird dort dann freigegeben und verstoffwechselt. Nach ausreichender Einwirkzeit wird das Papier entfernt und die Fluoreszenz aus der Tasche analysiert.

In weitere Abwandlung kann man Modifikationsmaterial auch direkt in unmittelbarer Nachbarschaft zum Tascheneingang plazieren. In diesem Fall erhält man bedingt durch das Konzentrationsgefälle der Modifikationssubstanz ein Hineinwandern desselben in die Tasche.

Wie oben dargelegt, steht am Anfang jeder Befunderhebung die Bestimmung der Taschentiefe. Diese Bestimmung führt zwangsläufig zu einer geringen Änderung des Tascheninhaltes. Will man eine solche Änderung vermeiden, so kann man zu einen die Bestimmung der Taschentiefe als letzten Schritt der Befunderhebung vorsehen, muß aber dann unter Umständen auf die Befunderhebung "Blutung auf Sondierung" und "Eiteraustritt" verzichten.

Aus dem diesem Grunde wird vorgeschlagen, die Messung der Taschentiefe und die Applikation des Modifikationsmateriales in einem Schritt zusammenzufassen. Hierzu versieht man eine Applikationskanüle zugleich mit einer Graduierung, welche die Taschentiefe zu messen gestattet. Dies wird nachstehend noch genauer beschrieben.

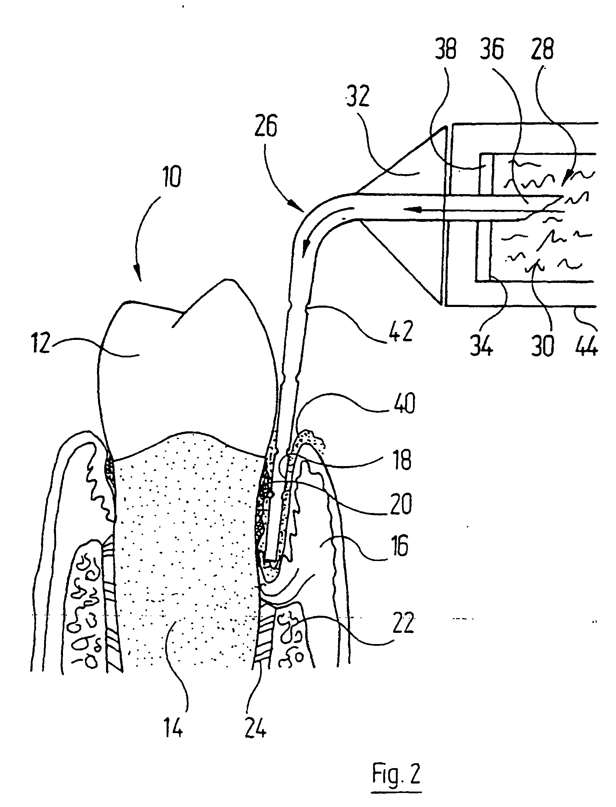

Nachstehend wird nun der apparative Aspekt der Erfindung näher erläutert, wobei auf die Figuren 2 bis 5 Bezug genommen wird.

In Figur 2 ist mit 10 ein Zahn bezeichnet, der eine Zahnkrone 12 und eine Zahnwurzel 14 aufweist. Mit 16 ist das den Zahn umgebende Zahnfleisch (Gingiva) bezeichnet. Am oberen Ende der Zahnwurzel 14 hat sich zwischen Zahnwurzel und Zahnfleisch 16 eine Zahnfleischtasche 18 gebildet. Auf dem Zahnhals hat sich eine Zahnsteinschicht 20 gebildet. Zwichen einem Abschnitt 22 des Kieferknochens und der Zahnwurzel liegt eine Schicht 24 aus Parodontalfasern.

In die Zahnfleischtasche 18 ist eine insgesamt mit 26 bezeichnete Kanüle eingeführt, die mit einer Kartusche 28 verbunden ist, in welcher ein Modifikationsmaterial 30 enthalten ist. Zur Verbindung mit der Kartusche 28 trägt die Kanüle 26 ein Befestigungsteil 32, welches mit dem mit 34 bezeichneten Kartuschengehäuse verbunden ist. Ein spitzer Endabschnitt 36 der Kanüle 26 ist durch eine Siegelwand 38 des Kartuschengehäuses 34 in das Modifikationsmaterial 30 eingeführt.

Durch manuelles Bewegen eines in der Zeichnung nicht wiedergegebenen Verdrängerkolbens kann man das Modifikationsmaterial 30 durch die Kanüle 26 in die Zahnfleischtasche 18 drücken. Eine ausgebrachte Menge an Modifikationsmaterial 30, die sich in der Zahnfleischtasche 18 befindet, ist in der Zeichnung mit 40 bezeichnet.

Positioniert man die Kanüle 26 so, daß ihr unteres Ende dem Taschenboden benachbart ist, so kann man an auf der Außenseite der Kanüle angebrachten Marken 42 gleichzeitig mit dem Einbringen des Modifikationsmateriales in die Zahnfleischtasche auch die Tiefe der Zahnfleischtasche messen.

Zum gleichmäßigen Füllen der ganzen Zahnfleischtasche 18 wird die Kanüle bezogen auf die Achse des Zahnes 10 in Umfangsrichtung und unter Aufrechterhaltung der achsparallelen Ausrichtung der Kanüle in der Zahnfleischtasche 18 bewegt, so daß letztere gleichzeitig gleichmäßig mit Modifikationmaterial gefüllt wird und bezüglich der Taschentiefe ausgemessen wird.

Falls gewünscht, kann man dem Modifikationsmaterial zugleich auch ein Röntgenkontrastmittel beifügen und nachdem Einbringen des Modifikationsmateriales die Kontur der Zahnfleischtasche 18 durch eine Röntgenaufnahme festhalten.

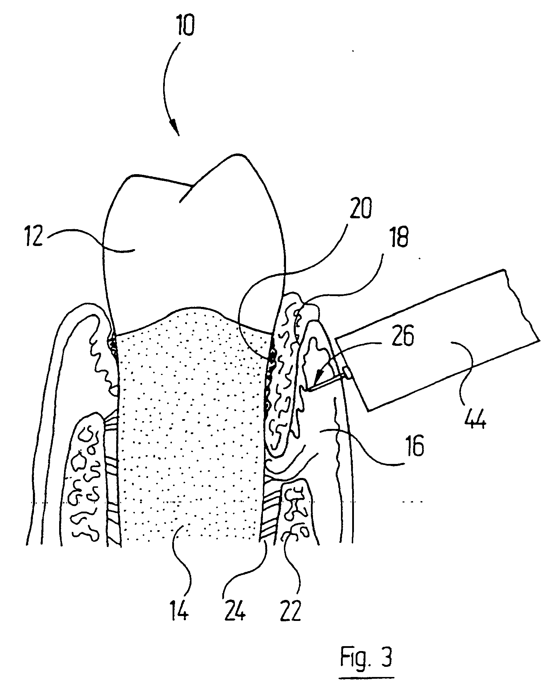

Alternativ kann man gemäß Figur 3 eine kurze dünne und scharfe Kanüle 26 in Verbindung mit einem flüssigen Modifikationsmaterial verwenden und durch das Zahnfleisch 16 hindurch das Modifikationsmaterial in die Zahnfleischtasche 16 oder in die dieser benachbarten Bereiche des Zahnfleisches 16 spritzen. Bei dieser Art des Vorgehens bleibt der Inhalt der Zahnfleischtasche 18 unverändert. Durch Diffusion gelangt die Modifikationssubstanz wieder an die Innenfläche der Zahnfleischtasche und an den Tascheninhalt. Das weitere Vorgehen nach dem Einspritzen des Modifiaktionsmateriales ist wieder gleich, wie oben beschrieben.

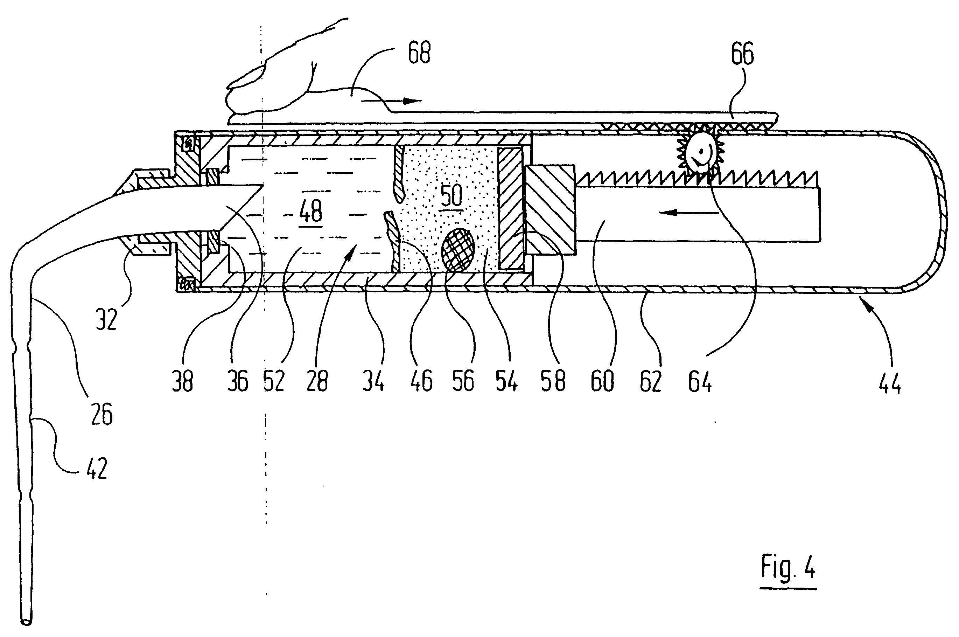

Figur 4 zeigt ein praktisches Ausführungsbeispiels eines Applikators 44 für höher Viskosität aufweisendes Modifikationsmaterial. Schon unter Bezugnahme auf Figur 2 angesprochene Teile sind wieder mit denselben Bezugszeichen versehen.

Die Kartusche 28 hat eine mittige zerstörbare Trennwand 46, welche den Innenraum der Kartusche in zwei Kammern 48, 50 unterteilt, von denen die eine (Kammer 48) das Grundmaterial 52, die andere (Kammer 50) die Modifikationssubstanz 54 enthält. In einer der Kammern (hier Kammer 50) ist eine Kugel 56 enthalten, welche zum Aufbrechen der Trennwand 46 durch Aufschlagen der Kartusche auf einer harten Fläche und zum vermischen des Inhaltes der beiden Kammern 48 und 50 dient, wie bei Zweikammer-Kartuschen üblich. Am hinteren Ende der Kartusche 28 ist ein Verdrängerkolben 58 wiedergegeben, der mit einer Zahnstange 60 verbunden ist. Diese ist über ein an einem Gehäuse 62 des Applikators gelagertes Ritzel 64 mit einer weiteren Zahnstange 66 gekoppelt, die in Längsrichtung des Applikatorgehäuses 62 bewegbar an diesem gelagert ist. Die Zahnstange 66 steht mit einem Betätigungsteil 68 in Verbindung.

Durch Schieben des Betätigungsteiles 68 in Figur 4 nach rechts nach aufbrechen der Trennwand 46 und vermischen des Inhaltes der beiden Kammern 48, 50 Modifikationsmaterial durch die Kanüle 26 ausgetragen.

In Abwandlung des oben beschriebenen Ausführungsbeispieles kann man das Betätigungsteil 68 auch direkt mit dem Verdrängerkolben 58 verbinden, wodurch die Betätigungsrichtung des Applikators 44 vertauscht wird.

Die Beobachtung der Fluoreszenz der kranken Zellen bzw. Bakterien kann im Prinzip dadurch erfolgen, daß man die Zahnfleischtasche 18 mit einem geeigneten Instrument lokal aufzieht, den gesamten Bereich der Zahnfleischtasche 18 mit dem Anregungslicht von 375 nm bis 440 nm beleuchtet und die rote Fluoreszenz direkt mit dem Auge über eine Beobachtungsfilter, welches im Bereich des roten Emissionspektrums des fluoreszierenden Protoporphyrins durchlässig ist, mit dem Auge betrachtet. Durch Bewegen des zum Abspreizen des Zahnfleisches verwendeten Werkzeuges in zirkulärer Richtung werden dann die verschiedenen Abschnitte der Zahnfleischtasche nacheinander inspiziert. Auf diese Weise erhält man ein Bild vom Ausmaß der Paradontitis und von der räumlichen Verteilung der kranken Bereiche. Das in dezentem Grün gesehene Hintergrundslicht erlaubt dabei die räumliche Zuordnung der fluoreszierenden Bereiche zur individuellen Gebißsituation.

Falls gewünscht kann man bei diesem direkten visuellen Betrachten der Fluoreszenz das im Roten durchlassende Beobachtungsfilter in ein Brillengestell einbauen, welches der Arzt trägt oder als Vorsatz vor eine Brille ausbilden. Alternativ kann man das Beobachtungsfilter an einem anderen Teil befestigen, z.B. dem zum partiellen Öffnen der Zahnfleischtasche verwendeten Werkzeug, einem etwa verwendeten Lichtleiter, über welchen das Anregungslicht zugeführt wird oder als elastisches Filter, welches sich unter Adaptation eines Lichtleiters elastisch an die Umgebung anpaßt und somit direkt an der Austrittsstelle des Fluoreszenzlichtes vorgesehen werden kann. Verwendet man zu Beobachtung eine Videokamera, kann das Beobachtungsfilter auf deren Objektiv aufgesetzt sein.

Zum einfachen Beleuchten und Betrachten des Inneren der Zahnfleischtasche kann man auch ein Diagnosegerät benutzen.

Bei den oben beschriebenen Ausführungsbeispielen wurde das Modifikationsmaterial dazu verwendet, erkrankte Bereiche über ihre optischen Eigenschaften zu markieren und so eine visuelle Kontrolle derselben zu ermöglichen. Das Modifikationsmaterial kann aber auch für Therapiezwecke eingesetzt werden. Man bringt das Modifikationsmaterial in irgendeiner seiner oben beschriebenen Realisierungsformen wieder an die kranken Stellen. Nach einer Einwirkungszeit von mindestens 60 bis 80 min, vorzugsweise etwa 120 min, sind die kranken Zellen photosensibilisiert, wie oben beschrieben wurde. Man kann nun diese photosensibilierten Zellen durch intensive Bestrahlung mit Licht so weit schädigen, daß sie absterben. Hierzu kann man z.B. Weißlicht mit einer Beleuchtungsstärke von 0,3 W/cm² über einen Zeitraum von 1 min einwirken lassen, wodurch schon eine signifikante Reduktion vitaler Bakterien in der Zahnfleischtasche erhalten wird, die über Zeiträume von mehreren Wochen anhält.

Durch eine solch intensive Bestrahlung mit Weißlicht werden auch gesunde Gewebebereiche beeinträchtigt, wenn auch nicht so geschädigt, daß sie absterben. Um derartige vorübergehende Gewebebeschädigungen zu vermeiden bzw. kleinzuhalten, kann man bei der Bestrahlung Maskenteile verwenden, die benachbarte gesunde Gewebebereiche abdecken.

Bei notwendiger längerfristiger Bestrahlung kann man die zu schützenden der Bestrahlungsstelle benachbarten Gewebebereiche zusätzlich durch ein feines Wasserspray oder durch Kühlluft kühlen, um zuweilen vom Patienten bei der photodynamischen Therapie wahrgenommene Schmerzen auszuschließen.

Die Verwendung sichtbaren Lichtes erfordert, daß ein freier Zugang zu dem zu bestrahlenden Bereich besteht. Ein solcher muß oft durch Abheben des Zahnfleisches vom Zahnhals geschaffen werden, was für den Patienten unangenehm ist. Wo ein solches Öffnen der Zahntaschen unerwünscht ist, kann man die photodynamische Therapie auch von der Seite durch die Gingiva hindurch vornehmen. Hierzu wird Licht verwendet, welches von der wasserhaltigen Gingiva nicht oder nur wenig absorbiert wird. Infrarotes Licht oder langwelligere elektromagnetische Strahlung wie Mikrowellen erfüllen diese Voraussetzung.

In weiterer Abwandlung der oben beschriebenen Ausführungsbeispiele kann man dem Modifikationsmaterial und/oder dem Koppelmedium weitere Substanzen zumischen, welche entweder den weiteren Verlauf der Untersuchung begünstigen (z.B. blutstillende Mittel) oder im Hinblick auf Prophylaxe und/oder medikamentöse Therapie von Vorteil sind. Zu nennen sind hier Fluoride, Desinfektionsmittel wie Chlorhexidin, desensibilisierende Substanzen wie Strontrium oder Kalium enthaltende Verbindungen, Antiadhäsiva wie Delmopinol oder Triclosan usw. Auf diese Weise kann man die für die Photosensibilisierung benötigte Zeit auch gleich zu Therapie- oder Prophylaxemaßnahmen nutzen.

Bei den oben beschriebenen Ausführungsbeispielen wurden kontinuierlich arbeitende Lichtquellen verwendet. Es versteht sich, daß man insbesondere im Rahmen der photodynamischen Therapie auch Blitz-Lichtquellen wie Stroboskopleuchten verwenden kann. Dabei kann im Rahmen der photodynamischen Therapie durch Einstellung des Tastverhältnisses zwischen Blitzzeit und Blitzabstand eine etwaige Schmerzreaktion vermindert werden und die Gefahr thermischer Schädigung umliegender gesunder Weich- und/oder Hartgewebe minimiert werden. Derartige Blitz-Lichtquellen können sowohl bei direkter Beleuchtung bzw. Bestrahlung oder auch bei Beleuchtung bzw. Bestrahlung über in die Tasche eingeführte Lichtabgabeelemente oder bei Beleuchtung oder Bestrahlung einer Mehrzahl von Zahntaschen durch eine reflektierende Verteilschiene, wie oben beschrieben, verwendet werden.

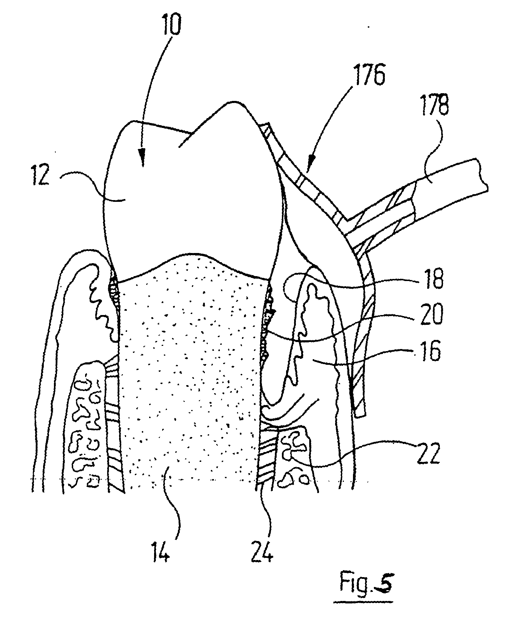

Gemäß Figur 5 kann man in eine Zahnfleischtasche 18 eingebrachtes Modifiziermaterial 40 dadurch gegen Mundflüssigkeit und gegen eine Abgabe von Modifikationssubstanz in den Mundraum schützen, daß man das ober offene Ende der Zahnfleischtasche gegen die Mundhöhle mit einer elastischen Schutzkappe 176 abdichtet. Diese kann mechanisch z.B. durch eine Drahtbügelfeder am Zahn 10 oder diesem benachbarten Zähnen angebracht werden, oder durch Unterdruck am Platz gehalten werden, wozu sie einen mit einer Unterdruckquelle verbindbaren Schlauch 180 aufweist.

Oben wurde die Erfindung unter Bezugnahme auf eine photodynamische Diagnostik und unter Bezugnahme auf eine photodynamische Therapie beschrieben. In der Praxis wird man diese Einsatzmöglichkeiten der Erfindung kombinieren mit anderen Paradontaltherapieverfahren, insbesondere mit einer Ultraschalltherapie.

Die erfindungsgemäße photodynamische Diagnostik empfiehlt sich parallel zu jeder herkömmlichen Paradontaldiagnostik, insbesondere bei Verdachtsbefunden einer etablierten marginalen Paradontitis. Nach Markierung aktiver Läsionen im Fluoreszenzkontrast, wie oben beschrieben, empfiehlt sich eine anschließende photodynamische Therapie, um die Entzündungskorrelate und ursächlichen Bakterien auch in für herkömmliche mechanische Verfahren oder für ein Ultraschalltherapieverfahren unzugänglichen Abschnitten der Zahnfleischtaschen zu erreichen. Unter Einsatz der Erfindung können somit erstmalig auch ins Taschenepithel und ins benachbarte Bindegewebe infiltrierte Bakterien unter vollständigem Verzicht auf Antibiotika und weitgehende Vermeidung systemischer Nebenwirkungen wirksam bekämpft werden.

Nach Abschluß der photodynmischen Therapie (und ggf. nach erneuter Diagnostik) wird anschließend eine herkömmliche mechanische Reinigung oder Spülung der betroffenen Paradontien durchgeführt, bevorzugt eine Ultraschalltherapie durchgeführt. Letztere hat den großen Vorteil, daß mit ihr auch eventuelle Gelrückstände und photodynamisch inaktivierte Bakterien besonders wirksam aus der Tasche entfernt werden. Auch etwa photodynamisch nicht inaktivierte Bakterien werden durch die ultraschallinduzierten Flüssigkeits-Scherbwegungen, durch Gravitation, durch Wärmeentwicklung oder (im Falle von an Zahn- oder anderen Gewebsoberflächen adhäsiv gebundenen Bakterien) durch mechanischen Abtrag wirkungsvoll entfernt.

Während der bei Paradontalbehandlungen notwendigen Befundreevaluation kommt die erfindungsgemäße Diagnostik erneut zum Einsatz, wodurch man die Therapieeffekte differenzierter und mit feinerer Auflösung beurteilen kann als mit den bisherigen diagnostischen Verfahren. Dies ist Voraussetzung für eine individuelle Planung der Erhaltunstherapie im Sinne einer (Re-)Infektionkontrolle und Paradontalprophylaxe.

Von der Erfindung kann man auch im Bereich der Diagnostik von Kariesläsion Gebrauch machen, insbesondere an verdeckten Stellen im Bereich der Fissurenkaries und der Approximalkaries.

Die Diagnostik erfolgt analog, wie obenstehend für die Paradontaldiagnostik beschrieben. Die Karies verursachenden Bakterien (Streptokokken) sind nämlich ebenfalls stoffwechselaktiv. Ihre Säureproduktion ist Ursache der Karies. Die betroffenen Hartsubstanzoberflächen weisen dagegen keinen Stoffwechsel (im hier relevanten Sinne) auf, weshalb sich gute Kontraste zwischen Bakterienschichten und Zahnoberfläche ergeben. Teilweise sind hier zwar noch Eigenfluoreszenzeffekte der Zahnhartsubstanz überlagert; diese spielen jedoch nur eine untergeordnete Rolle.

Die Applikation des Modifikationsmateriales muß wieder so erfolgen, daß unter den Umgebungsbedingungen die Modifikationssubstanz über eine zur Verstoffwechselung ausreichende lange Zeit an den fraglichen Bereichen der Zahnhartsubstanzoberflächen gehalten wird. Als Grundmaterial eignen sich daher besonders gut lösungsmittelhaltige Lacke, z.B. auf der Basis von Harzen oder Wachs, beispielsweise Kollophonium, Schellack etc. Bevorzugt werden hydrophile Substrate, welche auch unter Speichelzutritt über die zur Verstoffwechselung notwendige Zeit gut an den Zahnoberflächen haften.

Es können auch zunächst Modifikationsmaterialien, welche hydrophile Gele als Grundmaterial umfassen, aufgetragen werden, wie im Zusammenhang mit der Paradontaldiagnostik oben beschrieben wurde, z.B. Glyzeringel. Diese Materialien können dann zur Langzeitstabilisierung am betrachteten Ort mit einem anderen Material überschichtet werden, vorzugsweise einem Lack der oben angesprochenen Art.

Alternativ eignen sich als Grundmaterialien für die von den Bakterien zu verstoffwechselnden Modifikationssubstanzen oder zur Uberschichtung primär aufgetragener Modifikationsmaterialien auch Kunststoffe, z.B. Siloxane, Polyether, oder auch Materialien auf Agar-Basis. In Betracht kommt auch ein Schutz des aufgetragenen Modifikationsmaterials durch Anbringen eines Überabdrucks oder durch Aufsetzen einer Isolationsschiene, die das Modellmaterial vom Inneren des Mundraumes trennt.

Die oben beschriebene Verwendung der erfindungsgemäßen Diagnostik für die Karieserkennung erlaubt in kritischen Fällen die Identifizierung einer Initialkariesläsion, insbesondere nach zuvor erfolgter Oberflächenreinigung von adsorbierter Plaque von den betroffenen Zahnoberflächen. Dies ist darauf zurückzuführen, daß die Modifikationssubstanz und die Stoffwechselprodukte in Oberflächenrandzonen oder suboberflächliche Zahnhartsubstanzdefekte eindiffundieren, wodurch eine quantifizierbare Diagnostik von Struktureinbrüchen möglich ist. Man erhält so wertvolle Informationen als sicherere Basis für eine Therapieentscheidung. Die quantitative Auswertung der Fluoreszenz-Diagnostik erlaubt insbesondere bei Grenzflächen invasiver Indikationsstellung Verlaufskontrolle im Sinne eines Kariesmonitoring und ggf. auch die Kontrolle therapeutisch induzierter Stagnationen oder Remineralisationen. Auch in dieser Anwendung ist der Einsatz der Videotechnik mit geeigneten Filtern und nachgeordneter Bildauswertung vorteilhaft.

Anders als bei der Paradontalanwendung, bei welcher leicht aus der Zahnfleischtasche herauszuspülende "floating plaque" vorliegt, ist die kariogene Plaque fest an der Zahnoberfläche adhäriert. Zur besseren Infiltration der Modifikationssubstanz in Oberflächendefekte hat sich daher eine Applikation unter Verwendung einer geschlossenen Saugglocke oder einer fluiddicht über den zu untersuchenden Bereich gesetzten Formlöffels bewährt, wobei man dann das Penetrieren des Modifikationsmateriales in die Oberflächendefekte durch Wechseldruckbeaufschlagung des Inneren der Saugglocke oder des Abformlöffels unterstützen kann. Man erhält so eine Pumpwirkung, die durch positive und negative Druckspitzen herbeigeführt wird.

Mit besonderem Vorteil wird die erfindungsgemäße Kariesdiagnostik auch im Randbereich von zahnrestaurationen verwendet. Erkennt man dort eine beginnende Karies, so kann man durch ggf. durch Wechseldruckbeaufschlagung wie oben beschrieben unterstütztes Eindiffundieren eines Wirkstoffes in undichte und bakteriell besiedelte Randspalten eingegliederter Zahnrestaurationen die Karies im Anfangsstadium wirksam bekämpfen und eine Neuanfertigung der Zahnrestauration vermeiden. Insbesondere Leakage-Phänomene, die eine Neuanfertigung der Zahnrestauration langfristig notwendig machen, können mit der Fluoreszenz-Kariesdiagnostik zuverlässig erkannt werden.

## Patentansprüche

1. Verwendung eines Materials zur unterschiedlichen Modifizierung der optischen Eigenschaften unterschiedlicher Zellen mit einem Grundmaterial und einer in diesem verteilten Modifikationssubstanz bei der Herstellung eines Diagnostikums und/oder Therapeutikums für die Paradontologie und/oder Kariesdiagnostik, wobei das Grundmaterial eine viskose Flüssigkeit, ein Gel, ein flächiges oder dreidimensionales poröses Substrat oder ein in situ erhärtendes Material, insbesondere ein filmbildendes Material umfaßt und die Modifikationssubstanz die Menge an im Häm-Zyklus gebildetem Protoporphyrin erhöht, wobei die Modifikationssubstanz entweder ein Substrat für Enzyme, die nach der 5-Aminolävulinat-Synthase bis zum Schritt der Synthese des Protoporphyrins aktiv sind, oder ein Antagonist der Protoporphyrin abbauenden Ferro-Chelastase oder eine Eisen inaktivierende Substanz enthält.

2. Verwendung nach Anspruch 1, **dadurch gekennzeichnet, daß** das Grundmaterial bezüglich der unterschiedlichen Zellen und vorzugsweise auch bezüglich des Umgebungsmilieus inert ist.

3. Verwendung nach Anspruch 1 oder 2, **dadurch gekennzeichnet, daß** das Material thixotrop ist.

4. Verwendung nach einem der Ansprüche 1 bis 3, **dadurch gekennzeichnet, daß** das Grundmaterial ein Gel ist und mindestens einen feinen anorganischen Füllstoff umfaßt.

5. Verwendung nach einem der Ansprüche 1 bis 4, **dadurch gekennzeichnet, daß** das Grundmaterial ein Gel ist und ein organisches Verdickungsmittel umfaßt.

6. Verwendung nach Anspruch 1 oder 2, **dadurch gekennzeichnet, daß** das mit der Modifikationssubstanz versehene poröse Substrat in Pulverform vorliegt.

7. Verwendung nach einem der Ansprüche 1 bis 6, **dadurch gekennzeichnet, daß** das Grundmaterial transparent ist.

8. Verwendung nach einem der Ansprüche 1 bis 7, **dadurch gekennzeichnet, daß** das filmbildende Material einen Lack, ein Harz oder ein Wachs umfaßt.

9. Verwendung nach einem der Ansprüche 1 bis 8, **dadurch gekennzeichnet, daß** das Material zusätzlich eine Wirksubstanz enthält, z.B. ein Fluorid, ein Desinfektionsmittel, eine desensibilisierende Substanz oder ein Antiadhäsivum.

10. Verwendung nach einem der Ansprüche 1 bis 9, **dadurch gekennzeichnet, daß** das Material einen pH-Wert von 4, 5 bis 7,5, vorzugsweise etwa 5, aufweist.

11. Verwendung nach einem der Ansprüche 1 bis 10, **dadurch gekennzeichnet, daß** das Grundmaterial ein dentales Abformmaterial umfaßt.

12. Verwendung nach einem der Ansprüche 1 bis 11, **dadurch gekennzeichnet, daß** die Modifikationssubstanz eine die Fluoreszenz der Zellen modifizierende Substanz ist.

13. Verwendung nach einem der Ansprüche 1 bis 11, **dadurch gekennzeichnet, daß** die Modifikationssubstanz eine die Absorption der Zellen modifizierende Substanz ist.

14. Verwendung nach einem der Ansprüche 1 bis 13, **dadurch gekennzeichnet, daß** die Modifikationssubstanz 5-Aminolävulinsäure umfaßt.

15. Verwendung nach einem der Ansprüche 1 bis 14, **dadurch gekennzeichnet, daß** die Eisen inaktivierende Substanz einen Eisenkomplexbildner umfaßt.

16. Verwendung nach einem der Ansprüche 1 bis 15, **dadurch gekennzeichnet, daß** die Konzentration der Modifikationssubstanz 0,5 bis 50 Gew.-%, vorzugsweise zwischen 5 und 40 Gew.-%, wiederum bevorzugt 10 bis 20 Gew.-%, beträgt.

17. Gerät zum Applizieren eines viskosen Modifikationsmaterials nach einem der Ansprüche 1 bis 16, **dadurch gekennzeichnet, daß** es eine Kanüle (26) aufweist, die mit einer das Modifikationsmaterial unter Druck bereitstellenden Materialquelle (28, 58) verbunden ist.

18. Gerät nach Anspruch 17, **dadurch gekennzeichnet, daß** die Kanüle (26) eine auf ihre freie Spitze bezogene Graduierung (42) aufweist.

19. Gerät nach Anspruch 17 oder 18, **dadurch** gekennzeichet, daß die Kanüle (26) und die Materialquelle (28, 58) so ausgelegt sind, daß das Material langsam abgegeben wird.

20. Gerät nach Anspruch 17 oder 18, **dadurch gekennzeichnet, daß** die Kanüle (26) dünn und scharf ist.

21. Gerät nach einem der Ansprüche 17 bis 20, **dadurch gekennzeichnet, daß** die Materialquelle (28, 58) manuell bedienbar ist und zwischen einem manuell betätigten Bedienteil (68) und einem auf ein Volumen des Materials arbeitenden Verdrängerteil (58) ein die Bewegung des Bedienteils (68) untersetzendes Getriebe vorgesehen ist.

## Claims

1. Use of a material for differently modifying the optical properties of different cells with a basic material and with a modification substance distributed in the latter for the production of a diagnostic and/or therapeutic composition for periodontology and/or caries diagnosis, where the basic material comprises a viscous fluid, a gel, a two-dimensional or three-dimensional porous substrate or a material which hardens in situ, in particular a film-forming material, and where the modification substance increases the amount of protoporphyrin formed in the heme cycle, wherein the modification substance comprises either a substrate for enzymes which are active after 5-aminolevulinate synthase up to the step of protoporphyrin synthesis, or an antagonist of the protoporphyrin-degrading ferrochelastase or an iron-inactivating substance.

2. Use of claim 1, **characterized in that** the basic material is inert in relation to the different cells and preferably also in relation to the surrounding medium.

3. Use of claims 1 or 2, **characterized in that** the material is thixotropic.

4. Use of anyone of claims 1 through 3, **characterized in that** the basic material is a gel and comprises at least one fine inorganic filler.

5. Use of anyone of claims 1 through 4, **characterized in that** the basic material is a gel and comprises an organic thickener.

6. Use of claims 1 or 2, **characterized in that** the porous substrate provided with the modification substance is in powder form.

7. Use of anyone of claims 1 through 6, **characterized in that** the basic material is transparent.

8. Use of anyone of claims 1 through 7, **characterized in that** the film-forming material comprises a paintwork, a resin or a wax.

9. Use of anyone of claims 1 through 8, **characterized in that** the material additionally contains an active substance, for example a fluoride, a disinfectant, a desensitizing substance or an antiadhesive.

10. Use of anyone of claims 1 through 9, **characterized in that** the material has a pH from 4.5 to 7.5, preferably about 5.

11. Use of anyone of claims 1 through 10, **characterized in that** the basic material comprises a dental impression material.

12. Use of anyone of claims 1 through 11, **characterized in that** the modification substance is a substance modifying the fluorescence of the cells.

13. Use of anyone of claims 1 through 11, **characterized in that** the modification substance is a substance modifying the absorption of the cells.

14. Use of anyone of claims 1 through 13, **characterized in that** the modification substance comprises 5-aminolevulinic acid.

15. Use of anyone of claims 1 through 14, **characterized in that** the iron-inactivating substance comprises an iron complexing agent.

16. Use of anyone of claims 1 through 15, **characterized in that** the concentration of the modification substance is from 0.5 to 50 % by weight, preferably between 5 and 40 % by weight, further preferably from 10 to 20 % by weight.

17. Apparatus for the application of a viscous modification material of anyone of claims 1 through 16, **characterized in that** it has a cannula (26) which is connected to a material source (28, 58) which provides the modification material under pressure.

18. Apparatus of claim 17 **characterized in that** the cannula (26) has a graduation (42) related to its free tip.

19. Apparatus of claims 17 or 18, **characterized in that** the cannula (26) and the material source (28, 58) are designed in that the material is released slowly.

20. Apparatus of claims 17 or 18, **characterized in that** the cannula (26) is thin and sharp.

21. Apparatus of anyone of claims 17 through 20, **characterized in that** the material source (28, 58) can be operated manually, and a gear reducing the movement of the operating part (68) is provided between a manually actuated operating part (68) and a displacement part (58) operating on a volume of the material.

## Revendications

1. Utilisation d'un matériau pour une modification différenciante des propriétés optiques de différentes cellules, avec un matériau de base et une substance de modification distribuée dans celui-ci, lors de la fabrication d'un produit de diagnostic et/ou d'un produit thérapeutique pour la parodontologie et/ou le diagnostic de caries, utilisation selon laquelle le matériau de base comporte un liquide visqueux, un gel, un substrat poreux plat ou tridimensionnel ou un matériau durcissant *in situ,* en particulier un matériau filmogène, selon laquelle la substance de modification augmente la quantité de protoporphyrine formée dans le cycle de l'hème, et selon laquelle la substance de modification contient soit un substrat pour des enzymes qui sont actives après la synthéase du 5-aminolévulinate jusqu'à l'étape de la synthèse de la protoporphyrine, soit un antagoniste de la ferro-chélatase décomposant la protoporphyrine, soit une substance inactivant le fer.

2. Utilisation selon la revendication 1 , **caractérisée en ce que** le matériau de base est inerte du point de vue des différentes cellules et de préférence aussi du point de vue du milieu environnant.

3. Utilisation selon la revendication 1 ou 2, **caractérisée en ce que** le matériau est thixotrope.

4. Utilisation selon l'une des revendications 1 à 3, **caractérisée en ce que** le matériau de base est un gel et comprend au moins une charge inorganique fine.

5. Utilisation selon l'une des revendications 1 à 4, **caractérisée en ce que** le matériau de base est un gel et comporte un épaississant organique.

6. Utilisation selon la revendication 1 ou 2, **caractérisée en ce que** le substrat poreux doté de la substance de modification se présente sous forme de poudre.

7. Utilisation selon l'une des revendications 1 à 6, **caractérisée en ce que** le matériau de base est transparent.

8. Utilisation selon l'une des revendications 1 à 7, **caractérisée en ce que** le matériau filmogène comprend une laque, une résine ou une cire.

9. Utilisation selon l'une des revendications 1 à 8, **caractérisée en ce que** le matériau contient en plus une substance active, par exemple un fluorure, un désinfectant, une substance désensibilisatrice ou un antiadhésif.

10. Utilisation selon l'une des revendications 1 à 9, **caractérisée en ce que** le matériau présente un pH de 4,5 à 7,5, de préférence d'environ 5.

11. Utilisation selon l'une des revendications 1 à 10, **caractérisée en ce que** le matériau de base comprend un matériau de moulage dentaire.

12. Utilisation selon l'une des revendications 1 à 1 1 , **caractérisée en ce que** la substance de modification est une substance qui modifie la fluorescence des cellules.

13. Utilisation selon l'une des revendications 1 à 11, **caractérisée en ce que** la substance de modification est une substance modifiant l'absorption des cellules.

14. Utilisation selon l'une des revendications 1 à 13, **caractérisée en ce que** la substance de modification comprend de l'acide 5-aminolévulique.

15. Utilisation selon l'une des revendications 1 à 14, **caractérisée en ce que** la substance inactivant le fer comprend un complexant du fer.

16. Utilisation selon l'une des revendications 1 à 15, **caractérisée en ce que** la concentration de la substance de modification est de 0,5 à 50 % en poids, de préférence entre 5 et 40 % en poids, de façon encore préférée de 10 à 20 % en poids.

17. Appareil pour l'application d'un matériau de modification visqueux selon l'une des revendications 1 à 16, **caractérisé en ce qu'**il comporte une canule (26) qui est reliée à une source de matériau (28, 58) mettant à disposition le matériau de modification sous pression.

18. Appareil selon la revendication 17, **caractérisé en ce que** la canule (26) comporte une graduation (42) pratiquée sur son extrémité libre.

19. Appareil selon la revendication 17 ou 18, **caractérisé en ce que** la canule (26) et la source de matériau (28, 58) sont conçues de telle sorte que le matériau soit débité lentement.

20. Appareil selon la revendication 17 ou 18, **caractérisé en ce que** la canule (26) est mince et aiguë.

21. Appareil selon l'une des revendications 17 à 20, **caractérisé en ce que** la source de matériau (28, 58) peut être commandée manuellement et qu'un réducteur qui démultiplie le mouvement d'une pièce de commande (68) commandée manuellement est prévu entre la pièce de commande (68) et une pièce de refoulement (58) travaillant sur un volume du matériau.
